# EUROPEAN PATENT APPLICATION

(11) **EP 2 156 847 A1**
(43) Date of publication of application: **24.02.2010**
(21) Application number: 08290786.6
(22) Date of filing: 19.08.2008
(51) Int. Cl.: A61K 45/06, A61K 31/517, A61K 31/472, A61P 13/08, A61P 13/10

(54) **New combination of active ingredients containing an alpha1-antagonist and a PDE 4 inhibitor.**

(71) Applicant: Sanofi-Aventis, 75013 Paris (FR)
(72) Inventor: Lluel, Philippe, 31520 Ramnoville Saint Agne (FR); Palea, Stefano, 31400 Toulouse (FR); Sofeir, Maurice, 75013 Paris (FR)
(74) Representative: Kugel, Dominique

(57) **Abstract**

The subject of the present invention is a new combination of active ingredients consisting of alfuzosin and PDE 4 inhibitor, and pharmaceutical compositions containing them, for ameliorating and/or treating benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

## Description

The subject of the present invention is a new combination of active ingredients consisting of α₁-antagonists and PDE4 inhibitors; and pharmaceutical compositions containing them, as a combined preparation for simultaneous, separate or sequential use, for ameliorating and/or treating benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

The active ingredients constituting the combination are present in the free state or in the form of one their salts.

The α₁-antagonists can be chosen, in particular, from doxazosin, terazosin and alfuzosin in the form of an enantiomer or diastereoisomer or a mixture, in particular a racemic mixture, or a salt thereof, in particular alfuzosin hydrochloride. They are intended especially for treating benign hypertrophy of the prostate.

PDE 4 inhibitors are cyclic guanosine 3',5'-monophosphate specific phosphodiesterase type 4. The PDE 4 inhibitors can be chosen from rolipram, drotaverine, roflumilast, cilomilast and piclamilast, or a salt thereof.

Quite surprisingly and unexpectedly, the α₁-antagonist - PDE 4 inhibitor combination of the invention proved to be endowed with a synergistic activity of the two active ingredients in ameliorating and/or treating benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

The term urinary incontinence includes mixed-incontinence, urge-incontinence, stress-incontinence, functional incontinence and overflow incontinence.

The combinations according to the invention can be formulated in pharmaceutical compositions for administration to mammals, including man, which is an object of the present invention.

According to the invention, α₁-antagonists and PDE 4 inhibitors can be administered in the form of a salt.

These salts include for example salts with mineral acids such as hydrochloric acid, hydrobromic acid, sulfuric acid, nitric acid, phosphoric acid; salts with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid; or salts with acidic amino acids such as aspartic, and glutamic acid. Pharmacological acceptable salts are preferred.

According to another embodiment of the invention, the active ingredients of the combination are alfuzosin and drotaverine.

A further embodiment of the present invention is the combination wherein the active ingredients are alfuzosin hydrochloride and drotaverine hydrochloride.

A further embodiment of the present invention is the combination wherein the active ingredients are alfuzosin and drotaverine hydrochloride.

A further embodiment of the present invention is the combination wherein the active ingredients are alfuzosin hydrochloride and drotaverine.

In the text, which follows, the quantities of α₁-antagonists and of PDE 4 inhibitors are expressed as α₁-antagonists and PDE 4 inhibitors equivalents in non-salified, free form.

Advantageously, the combination of the invention comprises α₁-antagonists and PDE 4 inhibitors in a molar ratio (PDE 4 inhibitor/alfuzosin) of 0.6 to 800, preferably of 1 to 20.

The dose and frequency of administration of the medicament of the present invention are not particularly limited, and they may be appropriately chosen depending on conditions such as the body weight or age of a patient, severity and the like. Generally, a daily dose for oral administration may be administered once a day or several times a day as divided portions, or once in several days.

When the composition of the invention is administered in man by the parenteral or oral route, it is preferable that the daily dose of α₁-antagonists is between 0.5 to 25 mg, the daily dose of PDE 4 inhibitors being between 15 to 250 mg.

When the composition of the invention is administered in adult man by the parenteral or oral route, the daily dose of drotaverine may vary between 15 to 250 mg. Preferably the daily dose of drotaverine may vary between 120 to 240 mg. More particularly, the dose of drotaverine hydrochloride can be 40 mg once to six times per day, or 80 mg, once to three times per day.

When the composition of the invention is administered in adult man by the parenteral or oral route, the daily dose of alfuzosine may vary between 2 to 15 mg. Preferably the daily dose of alfuzosine may vary between 2 to 10 mg. More particularly, the dose of alfuzosine hydrochloride can be 2.5 mg once to three times per day, or 5 mg once to two times per day, or 10 mg once per day.

In the pharmaceutical compositions of the present invention, the active ingredients are generally formulated in dosage units containing from 0.1 to 25 mg of the α₁-antagonist more particularly from 2 to 10 mg or from 2.5 to 10mg for the α₁-antagonist alfuzosin hydrochloride per unit dosage and from 15 to 80 mg of the PDE 4 inhibitor and more particularly from 20 to 80 mg of e.g. the PDE 4 inhibitor dotraverine hydrochloride per unit dosage.

One other object of the present invention is a pharmaceutical composition, which contains, as active ingredients, an α₁-antagonist and a PDE 4 inhibitor as a combined preparation for simultaneous use.

A further object of the present invention is a pharmaceutical composition, which contains, as active ingredients an α₁-antagonist and a PDE 4 inhibitor, as a combined preparation for separate or sequential use.

These compositions are preferably made so as to be administered by the oral or parenteral route.

In the pharmaceutical compositions of the present invention for oral, sublingual, subcutaneous, intramuscular, intravenous, intradermal, local or rectal administration, the active ingredient may be administered in unit forms for administration, mixed with conventional pharmaceutical carriers, to animals and to human beings. For example, the pharmaceutical composition may be formulated, for example, in the form of pharmaceutical compositions for oral administration such as granules, fine granules, powders, hard capsules, soft capsules, syrups, emulsions, suspensions, solutions and the like, or in the form for sublingual a buccal administration, or in the form of pharmaceutical compositions for parenteral administrations such as injections for intravenous, intramuscular, or subcutaneous administration, drip infusions, transdermal preparations, transmucosal preparations, nasal drops, inhalants, suppositories and the like. Injections or drip infusions may be prepared as powdery preparations such as in the form of lyophilized preparations, and may be used by dissolving just before use in an appropriate aqueous medium such as physiological saline. Sustained-release preparations such as those coated with a polymer may be directly administered intracerebrally.

Types of pharmaceutical additives used for the manufacture of the pharmaceutical composition, content ratios of the pharmaceutical additives relative to the active ingredient, and methods for preparing the pharmaceutical composition may be appropriately chosen by those skilled in the art. Inorganic or organic substances or solid or liquid substances may be used as pharmaceutical additives. Generally, the pharmaceutical additives may be incorporated in a ratio ranging from 1% by weight to 90% by weight based on the weight of an active ingredient.

Examples of excipients used for the preparation of solid pharmaceutical compositions include, for example, lactose, sucrose, starch, talc, cellulose, dextrin, kaolin, calcium carbonate and the like. When a solid composition in the form of tablets is prepared, the active ingredients are mixed with the excipients. The tablets can be coated with sucrose or other appropriate materials or alternatively they can be treated such that they have a prolonged or delayed activity and that they have a prolonged or delayed activity and that they continuously liberate a predetermined quantity of active ingredient.

A preparation in the form of gelatin capsules is obtained by mixing the active ingredient with a diluent and by pouring the mixture obtained into soft or hard gelatin capsules. For the preparation of liquid compositions for oral administration, a conventional inert diluent such as water or a vegetable oil may be used. The liquid composition may contain, in addition to the inert diluent, auxiliaries such as moistening agents, suspension aids, sweeteners, aromatics, colorants, and preservatives. The liquid composition may be filled in capsules made of an absorbable material such as gelatin. Examples of solvents or suspension mediums used for the preparation of compositions for parenteral administration, e.g. injections, suppositories, include water, propylene glycol, polyethylene glycol, benzyl alcohol, ethyl oleate, lecithin and the like. Examples of base materials used for suppositories include, for example, cacao butter, emulsified cacao butter, lauric lipid, witepsol.

It will be noted that according to the invention, α₁-antagonists and PDE 4 inhibitors can both be administered by the oral route, or both by the parenteral route or one can be administered by the oral route (preferably alfuzosin) and the other by the parenteral route (preferably PDE 4 inhibitors).

According to another of its objects, the present invention also relates to the use of a combination according to the invention, for the preparation of a medicament intended for the treatment or amelioration of benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

A further object of the present invention is a method for treating/ameliorating the pathologies indicated above, which comprises the administration to a patient of an effective amount of the combination according to the invention.

The combinations of active ingredients according to the invention are the subject of pharmacological studies.

For example the effect of a combination, according to the present invention, on human detrusor contractions can be tested according to European Urology 51 (2007) 772-781.

The following example is an illustration of the present invention.

### Example

A unitary form of administration of a pharmaceutical composition according to the invention in the form of a tablet can comprised the following:

| | |
|---|---|
| Alfuzosin hydrochloride | 10 mg |
| Drotaverine hydrochloride | 80 mg |
| Sodic croscaramellose | 0.6 mg |
| Corn starch | 1.4 mg |
| Hydroxypropyl-methylcellulose | 0.25 mg |
| Magnesium stearate | 0.35 mg |

A unitary form of administration of a pharmaceutical composition according to the invention in the form of a tablet can comprised the following:

| | |
|---|---|
| Alfuzosin hydrochloride | 5 mg |
| Drotaverine hydrochloride | 40 mg |
| Sodic croscaramellose | 0.6 mg |
| Corn starch | 1.4 mg |
| Hydroxypropyl-methylcellulose | 0.25 mg |
| Magnesium stearate | 0.35 mg |

## Claims

1. Pharmaceutical composition containing a combination of active ingredients, in which the active ingredients are an α₁-antagonist and a PDE 4 inhibitor and both active ingredients being present in the free state or in the form of a salt.

2. Pharmaceutical composition according to claim 1, in which the α₁-antagonist is chosen from doxazosin, terazosin and alfuzosin, or a salt thereof.

3. Pharmaceutical composition according to claim 1 or 2, in which the α₁-antagonist is alfuzosin, or a salt thereof.

4. Pharmaceutical composition according any one of the preceding claims, in which the PDE 4 inhibitor is chosen from rolipram, drotaverine, roflumilast, cilomilast and piclamilast, or a salt thereof.

5. Pharmaceutical composition according to any one of the preceding claims, in which the active ingredients are alfuzosin and drotaverine, each being present in the free state and/or in the form of a salt.

6. Pharmaceutical composition according to any one of the preceding claims, in which the active ingredients are alfuzosin hydrochloride and drotaverine hydrochloride.

7. Pharmaceutical composition according to any one of the preceding claims, in a form being able to be administered by the parenteral route or by the oral route.

8. Pharmaceutical composition according to any one of the preceding claims **characterised in that** α₁-antagonists and PDE 4 inhibitor are of molar ratio of 0.6 to 800.

9. Pharmaceutical composition according to any one of the preceding claims, for ameliorating and/or treating benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

10. Pharmaceutical composition according to any one of the preceding claims, as a combined preparation of α₁-antagonists and PDE 4 inhibitor for simultaneous use.

11. Pharmaceutical composition according to any one of the preceding claims, as a combined preparation of α₁-antagonists and PDE 4 inhibitor for separate or sequential use.

12. Use of a combination according to any one of the preceding claims, for the preparation of a medicament intended for the treatment or amelioration of benign prostatic hyperplasia, urinary disorders, disorders related to bladder dysfunction, lower urinary tract symptoms associated or not associated with benign prostatic hyperplasia, urinary incontinence, bladder outlet obstruction associated or not associated with benign prostatic hyperplasia, interstitial cystitis and overactive bladder.

13. Use according to claim 12, in which the treatment involves the administration by the parenteral and/or oral route of 0.5 to 15 mg of alfuzosin per day and of 15 to 80 mg of PDE 4 inhibitor per day.

14. Use according to claim 13, in which the PDE 4 inhibitor is chosen from rolipram, drotaverine, roflumilast, cilomilast and piclamilast, or a salt thereof.

15. Use according to claim 13 or 14, in which the PDE 4 inhibitor is drotaverine, or a salt thereof.
